(19) Europäisches Patentamt
European Patent Office
Office européen des brevets

(11) Publication number: 0 127 274
A2

(12) # EUROPEAN PATENT APPLICATION

(21) Application number: 84301874.8

(22) Date of filing: 20.03.84

(51) Int. Cl.³: C 07 D 233/94
A 61 K 31/415

(30) Priority: 25.03.83 US 478961

(43) Date of publication of application:
05.12.84 Bulletin 84/49

(84) Designated Contracting States:
BE CH DE FR GB IT LI NL SE

(71) Applicant: THE UPJOHN COMPANY
301 Henrietta Street
Kalamazoo, Michigan 49001(US)

(72) Inventor: Cho, Moo Jung
c/o The Upjohn Company 301 Henrietta Street
Kalamazoo Michigan 49001(US)

(74) Representative: Perry, Robert Edward et al,
GILL JENNINGS & EVERY 53-64 Chancery Lane
London WC2A 1HN(GB)

(54) **Esters of metronidazole and compositions containing them.**

(57) Amino-acid esters of metronidazole, in acid salt form, which have high solubility, allowing metronidazole to be formulated into parenteral dosage forms for a single bolus injection.

EP 0 127 274 A2

# ESTERS OF METRONIDAZOLE AND COMPOSITIONS CONTAINING THEM

This invention relates to metronidazole esters and, in particular, to amino-acid esters of metronidazole. The invention relates also to compositions containing the esters.

Metronidazole, i.e. 1-(β-hydroxyethyl)-2-methyl-5-nitroimidazole, is a known antibiotic used for the treatment of anaerobic infections, particularly *Trichomonas vaginalis*. It and its preparation are described in US-A-2944061.

Owing to its low solubility in water (approximately 10 mg/ml) relative to the therapeutically effective dose (250-500 mg), metronidazole is suitable only for prolonged intravenous infusion, and cannot be used for a single intravenous or intramuscular injection. US-A-4160827 discloses a phosphate ester of metronidazole which overcomes this solubility problem. However, the phosphate ester appears to decrease the bioavailability of the compound in humans. US-A-4032645 discloses an injectable metronidazole composition employing a buffer solution. However, this composition has a very low pH, making it unsuitable for injection.

The thesis title "Glycine metronidazole as a watersoluble prodrug" was catalogued and available to inspection by the public at Kalamazoo College, Michigan, USA, in January 1983. No further information was available.

Compounds according to the present invention are of the formula

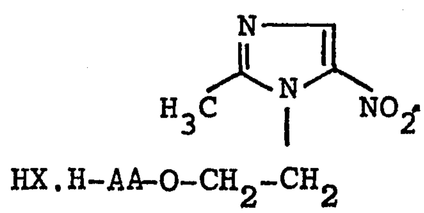

wherein AA represents that part of a naturally-occurring

amino-acid having at least 3 carbon atoms, of the formula H-AA-OH; and HX is an organic acid, an inorganic acid or anti-bacterial agent which includes an acidic group.

A pharmaceutical composition according to the invention is adpated for parenteral administration and comprises a compound of the invention and a pharmaceutical excipient.

Examples of suitable naturally-occurring amino-acids of the formula H-AA-OH are alanine (in which case AA is Ala, using conventional nomenclature), valine, leucine, isoleucine, phenylalanine and lysine (all preferred in this invention), and also proline, tryptophan, methionine, serine, threonine, cysteine, tyrosine, asparagine, glutamine, aspartic acid, glutamic acid, arginine and histidine.

Examples of HX include hydrochloric, hydrobromic, hydroiodic, sulfuric, phosphoric, lactic, maleic, malic, succinic, tartaric, 1-naphthalenesulfonic, 2-naphthalenesulfonic and ($C_{1-16}$ alkyl)carboxylic (e.g. acetic and propionic) acids, sulfonamides containing carboxyl groups such as succinylsulfathiazole and phthalylsulfathiazole, penicillins which contain a carboxyl function such as penicillin G, penicillin V, phenethicillin, methicillin, oxacillin, nafcillin, ampicillin, carbenicillin and ticarcillin, and cephalosporins such as cephalothin and cephaloridine. HCl is the preferred acid.

By "pharmaceutical excipient" is meant any of the additives known to the pharmaceutical art which are used to formulate parenteral compositions such as solutions and suspensions. The choice of additive depends on the objective. These excipients include buffer components, inorganic salts (frequently added to parenteral formulations to maintain tonicity) and pharmaceutical preservatives such as benzyl alcohol.

The parenteral formulation may be prepared in advance, and preserved in solution, or it may be packaged

in a two-component system consisting of a freeze-dried drug powder and a sterile diluent which are combined immediately prior to administration. The selection of suitable excipients, and the formulation of the compounds of the present invention into parenteral compositions, are readily undertaken by one ordinarily skilled in drug formulation.

The compounds of the present invention are useful in the same manner as metronidazole, e.g. as described in US-A-2944061 and particularly for the treatment of anaerobic infections. For this purpose, the compounds are administered intravenously, intramuscularly, topically, orally or buccally to humans and animals. Intravenous and intramuscular administration are preferred. A single dose is, for example, from 1 to 30 mg per kg.

The compounds of the present invention may be prepared by either of two routes. In route A, metronidazole (A-1) is reacted with a t-butyloxycarbonylamino-acid of the formula t-Boc-AA-OH (A-2) in the following manner. The metronidazole and N,N-dimethylaminopyridine are dissolved in dry pyridine. Separately, dicyclohexylcarbodiimide (DCC) and the t-butyloxycarbonylamino-acid are dissolved in pyridine. These two pyridine solutions are slowly mixed together, with continuous stirring. Dicyclohexylurea (DCHU) is formed and is filtered off. After the solvent has evaporated, the desired product has the formula A-3

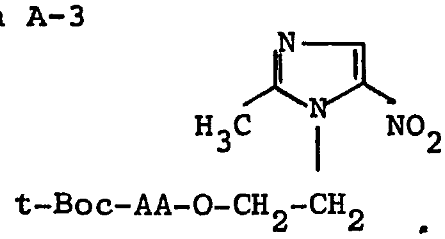

The formula A-3 compound is reacted with an acid of the formula HX to yield the compound of the invention.

Route B is a "mixed-anhydride" route. A t-butyloxycarbonylamino-acid (A-2) and N-methylmorpholine

are dissolved in dry tetrahydrofuran (THF). To this solution, isobutyl chloroformate is added dropwise under a nitrogen atmosphere. After the precipitates which form are filtered off, a mixture of metronidazole of the Formula A-1 and dimethylaminopyridine (DMAP) in dry THF is added. After 60 minutes at room temperature, the reaction mixture is dried under vacuum. If necessary, the pure product of the Formula A-3 is isolated by means of liquid chromatography. The final product is then prepared as described above.

The amino-acid esters of metronidazole synthesized herein are useful for parenteral administration of the drug. A single bolus parenteral dosage form has not been possible using metronidazole itself because of the poor intrinsic solubility of metronidazole in water. The aqueous solubility of the amino-acid esters of metronidazole is, however, sufficiently high at a pH below the pKa of the alpha amino group (approximately 7.4) such that a single dose can be formulated having a total volume of 1.0 to 2.0 ml.

Further, the ester compounds of the present invention have improved bioavailability as compared to metronidazole phosphonate. As set out more fully in Example 3, some of the compounds have shorter half-lives and higher rate constants than others in the hydrolysis back to the parent compound (metronidazole) and amino-acid. These compounds would thus tend to rather quickly attain higher blood levels of metronidazole. On the other hand, those compounds with longer half-lives would tend to be more useful for sustained action. Thus, the choice of which compound to use depends on the objective to be achieved. Thus, based on Example 3, the phenylalanine ester is preferred if one wishes to quickly obtain high blood levels of metronidazole, while the valine or isoleucine esters are preferred for a long duration of action.

The properties of the amino-acid esters of metronidazole claimed herein, as described above, make them suitable for a single, bolus, parenteral injection which is not possible with any other known metronidazole composition. Most importantly, the compounds and compositions of the present invention are suitable for use in a single bolus intravenous or intramuscular injection. These modes of administration are particularly useful for antibiotics such as metronidazole. These compounds are also useful for prolonged intravenous infusion like metronidazole itself, although the bolus

injection is preferred.

The following Examples 1 and 2 illustrate the preparation of the invention. Example 3 illustrates their utility. TFA is trifluoroacetic acid. "Lobar", "miniPump", Dowex" and "Minipulse" may be registered Trade Marks.

Example 1 Acid salts of Alanine, Valine, Leucine, Phenylalanine, and Lysine Esters of Metronidazole

For each amino-acid ester, 30 mmole of DCC (6.19 gm) and a Boc derivative of amino-acid in the quantity tabulated below are dissolved in 40 ml of dry pyridine. After 15 to 30 minutes at room temperature, DCHU is filtered off, and the filtrate is added to 40 ml of pyridine containing 10 mmole (1.72 gm) of metronidazole and 0.5 mmole (0.061 gm) of DMAP. The reaction is allowed to proceed at room temperature for 60 minutes, concentrated under vacuum, and the residue is taken up into 100 ml ethyl acetate. The ethyl acetate solution is then washed successively with 100 ml of 0.1 M formate buffer at pH 4.0 and 100 ml of 0.1 M phosphate buffer at pH 7.6 or 100 ml of 0.1 M carbonate buffer at pH 9.0. These buffers used for washing the ethyl acetate solution are all pre-saturated with sodium chloride. After being washed with water, the ethyl acetate layer is dried with sodium sulfate and concentrated to dryness under vacuum.

Residues containing the Boc-derivatives, with the exception of Boc-alanine ester, are further purified by means of the preparative liquid chromatography (LC) procedure described below. The Boc-alanine ester needs no further purification. Reaction mixtures are loaded on two commercially obtained LC columns in series (Merck Lobar ; size B and C), and eluted with a mobile phase consisting of n-hexane/ethyl acetate/methanol/formic acid (50:47:2:1) at a flow rate of 8.0 ml/minute (Milton Roy miniPump Model NSI-33R). Eluent is continuously monitored at a given wave-length (Gilford Model 250 spectrophotometer) by use of a flow-through cell. Fractions containing products are combined and evaporated to dryness.

All Boc-derivatives are treated with 50 ml of 25%-trifluoroacetic acid (TFA; Aldrich) in dry methylene chloride, and stirred for 60 minutes. The excess TFA and methylene chloride are then removed by evaporation under a reduced pressure at 60 C. The Boc-valine ester requires more strenuous conditions. 75% TFA is used and the reaction is allowed to continue overnight.

The TFA salt of phenylalanine, lysine or alanine ester of metronidazole is converted to chloride salt by an anionic exchange column chromatography; Dowex 2-X8 (25 ml of wet volumn in $Cl^-$ form). A Gilson Model Minipulse II provided a flow rate of 1.5 ml/min. The eluent is monitored at 340 nm. The fractions containing the product are combined and dried. If required, the chloride salts are crystallized in isopropanol. The overall yield for an analytical sample is in the range of 5 to 10%.

The physical characteristics of the products are as follows: Alanine Ester: $R_f$=0.53 (methylene chloride/isopropanol/methanol/28.4% ammonium hydroxide (80:15:4:1)); mp=140-42C; NMR ($CD_3OD$, δ) 1.4, 2.6, 4.2, and 4.8. Anal. Calc'd. for $C_{11}H_{14}O_4N_4Cl$: C, 38.89; H, 5.43; N, 20.15; Cl, 12.75. Found: C, 38.94; H, 5.55; N, 20.32; Cl, 12.82.

Phenylalanine Ester: $R_f$=0.77 (sec-butanol/pyridine/acetic acid/water (37.5:25:7.5:30)); mp=ca. 70C; NMR ($CD_3OD$), δ) 2.6, 3.4, 4.3, 4.8, 7.2, and 8.8 IR(KBr) 2900 $cm^{-1}$ and 1750 $cm^{-1}$.

Lysine Ester: $R_f$=0.58 (sec-butanol/pyridine/acetic acid/water (37.5:25:7.5:30)); mp=ca. 70C; NMR ($D_2O$, δ) 1.8, 2.6, 3.0, 4.1, 4.8, and 8.1 IR(KBr) 2950 $cm^{-1}$ and 1750$cm^{-1}$.

The TFA salt of the valine or leucine ester is dissolved in 100 ml of 0.1 M Tris buffer at pH 8.9, and the free base is extracted into 100 ml of ethyl acetate. After the solvent is evaporated, the residue is taken up into 10 ml of water and titrated to pH 5.7 with 0.1N HCl. After the water is evaporated, the residue is crystallized from isopropanol. The overall yield is in the order of 15 to 20%.

The physical characteristics of the products are as follows:

Valine Ester: $R_f$=0.73 (methylene chloride/isopropanol/methanol/-28.4% ammonium hydroxide (80:15:4:11)); MP=ca. 70C; NMR ($CD_3OD$, δ) 1.2, 2.2, 2.6, 4.0, 4.8, and 8.1. Anal. Calc'd. for $C_{11}H_{19}O_4N_4Cl$: C, 43.07; H, 6.24; N, 18.26; Cl, 11.56. Found: C, 41.90; H, 6.22; N, 18.03; Cl, 11.46.

Leucine Ester: $R_f$=0.83 (methylene chloride/isopropanol/-methanol/28.4% ammonium hydroxide (80:15:4:11)) mp=140-43C (dec.); NMR ($CD_3OD$, δ) 1.0, 2.8, 2.9, 4.2, 4.8, and 8.7. Anal. Calc'd. for $C_{12}H_{21}O_4N_4Cl$: C, 44.93; H, 6.60; N, 17.47; Cl, 11.05. Found: C, 45.13; H, 6.78; N, 17.43; Cl, 11.07.

Quantity of Boc-Amino-Acid Used in the Synthesis (Per 10 mmole of Metronidazole)

| Boc-Amino acid | mmole (gm) |
|---|---|
| Boc-Alanine | 27 (5.07) |
| Boc-Valine | 40 (8.69) |
| Boc-Leucine | 23 (5.24) |
| Boc-Phenylalanine | 40 (10.60) |
| Bis-Boc-Lysine | 40 (13.9) |

Example 2     Acid salt of Isoleucine Ester of Metronidazole

10 mmole of Boc-isoleucine (2.31 gm) and 10 mmole of N-methylmorpholine (Aldrich; 1.10 ml) are dissolved in 40 ml of dry tetrahydrofuran (THF). Under a nitrogen atmosphere, 10 mmole of isobutylchloroformate (Aldrich, 1.31 ml) is introduced to the system. After 3 minutes, the precipitate which forms is filtered off. Into the filtrate, 10 mmole of metronidazole (1.72 gm) and 0.5 mmole of DMAP (0.061 gm) in 40 ml of THF are added. After 60 minutes at room temperature, the reaction mixture is evaporated to dryness under vacuum. The residue is then taken up into 100 ml of ethyl acetate. It is washed with 100 ml of 0.1M formate buffer at pH 4.0 and 100 ml of 0.1M phosphate buffer at pH 8.0. After being washed with 75 ml of water, the ethyl acetate layer is dried over sodium sulfate. After the solvent is removed, the remaining residue is subjected to an LC separation. Two size B Lobar columns (Merck) are eluted at a flow rate 7.6 ml/min with a mobile phase composed of acetic acid/methanol/ethyl acetate/n-hexane (2:4:44:50). The eluent is continuously monitored at 355 mm. Fractions containing product are combined and evaporated to dryness.

Deprotection of the Boc-group is carried out in 75% TFA in methylene chloride overnight at room temperature. The excess TFA and the solvent is removed under a reduced pressure at 60° C. The TFA salt obtained is converted to the chloride salt through the extraction of the free base followed by titration with 0.1N-HCl. The chloride salt is recrystallized from isopropanol, and has the following characteristics: $R_f$=0.77 (methylene chloride/isopropanol/methanol/-28.4% ammonium hydroxide (80:15:4:11); mp=197-99°; NMR ($CD_3OD$, δ) 1.2, 2.1, 2.8, 4.1, 4.8, and 8.5. Anal. Calc'd. for $C_{12}H_{20}O_4N_4Cl$: C, 45.07; H, 6.30; N, 17.52; Cl, 11.09. Found: C, 44.76; H, 6.61; N, 17.46; Cl, 10.90.

Example 3     Serum Hydrolysis of Metronidazole Esters

The serum hydrolysis of the metronidazole esters prepared above was measured in an <u>in vitro</u> test as follows: After the blood from human volunteers was allowed to coagulate at RT for 60 minutes, the whole blood was centrifuged at 1000xg for 20 minutes at 4 C. The supernatant was centrifuged a second time at 3000xg for 20 minutes at 4 C. The resulting serum was decanted into fractions of about 10 ml and stored frozen at -10 C until use.

The hydrolysis was initiated by adding an aqueous solution of an ester to a given volume of serum which had been equilibrated at 37 C. In all cases, dilution of serum can be neglected. The initial substrate concentration was approximately 1 mg/ml except in the case of valine and isoleucine esters where 0.5 mg/ml was used. At a proper time interval, 0.5 ml of serum was withdrawn and injected to 2.0 ml of methanol. The solution was vortexed and the precipitated proteins were centrifuged off. The supernatant was diluted five times in a volumetric flask (usually 1.0 ml to 5.0 ml) with an appropriate mobile phase.

Using a constant-volume loop (Rheodyne Model 7000), 50 µl of the sample solution prepared as described above were injected to a reverse phase column (C-18, 10µ; Water Assoc.) coupled with an RP-18 pre-column (Rainin). Mobile phases used were; 0.02M citrate buffer at pH 6.0 containing 0.02M dimethyloctylamine (DMOA: Ethyl Corp.) for lysine ester, a 1:9 mixture of methanol:0.02M citrate buffer at pH 6.0 with 0.02M DMOA for alanine ester, a 1:3 mixture of methanol:0.02M citrate buffer at pH 5.0 with 0.02M DMOA for phenylalanine ester, and a 1:1 mixture of methanol:0.05M phosphate buffer at pH 6.7 containing sodium dodecylsulfate at 50 mg/l for valine and isoleucine esters. Other conditions for the HPLC assay were; DuPont Model 837 spectrophotometer detector, ALTEX Model 110A pump, λ=317 mm, FR=2.0 ml/min at P=2000~3000 psi, and AUFC determined by a Hewlett-Packard Model 3380A integrator.

The hydrolysis was followed by monitoring disappearance of the amino-acid ester of metronidazole in an aqueous buffer at 25 ±0.3C. The final ionic strength, in all cases adjusted to 0.10 M, was 0.01 M from sodium chloride and 0.01 M from the buffer components. The buffers used were: HCl, acetate, succinate, phosphate, Tris, carbonate, butylamine, and sodium hydroxide. The initial concentration of the ester was approximately 0.07 mg/ml in all cases.

The remaining metronidazole ester was determined by means of reverse-phase HPLC; see the above for the HPLC conditions. In general, the hydrolysis was monitored for at least two half-lives, and followed first-order kinetics in all cases. The hydrolysis rate constant ($k_{obs}$) was calculated from $K_{obs} \cdot t_{1/2} = \ln 2$.

The results are depicted in Figures 1 and 2. Results for the corresponding glycine ester salt are also shown.

CLAIMS

1. A compound of the formula

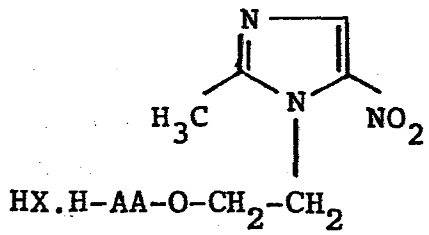

wherein AA represents that part of a naturally-occurring amino-acid having at least 3 carbon atoms, of the formula H-AA-OH; and HX is an organic acid, an inorganic acid or an anti-bacterial agent which contains an acidic group.

2. A compound as claimed in claim 1, wherein the amino-acid is selected from alanine, valine, leucine, isoleucine, phenylalanine and lysine.

3. A compound as claimed in claim 1 or claim 2, wherein HX is selected from hydrochloric, hydrobromic, hydroiodic, sulfuric, phosphonic, acetic, propionic, lactic, maleic, malic, succinic and tartaric acids, cephalosporins containing an acid function, and penicillins containing an acid function.

4. A compound as claimed in claim 1, wherein HX is hydrochloric acid.

5. Any of the six compounds as claimed in claim 2, wherein HX is hydrochloric acid (which is a dihydrochloride when the amino-acid is lysine).

6. A pharmaceutical composition, adapted for parenteral administration, which comprises a compound as claimed in any preceding claim and a pharmaceutical excipient.

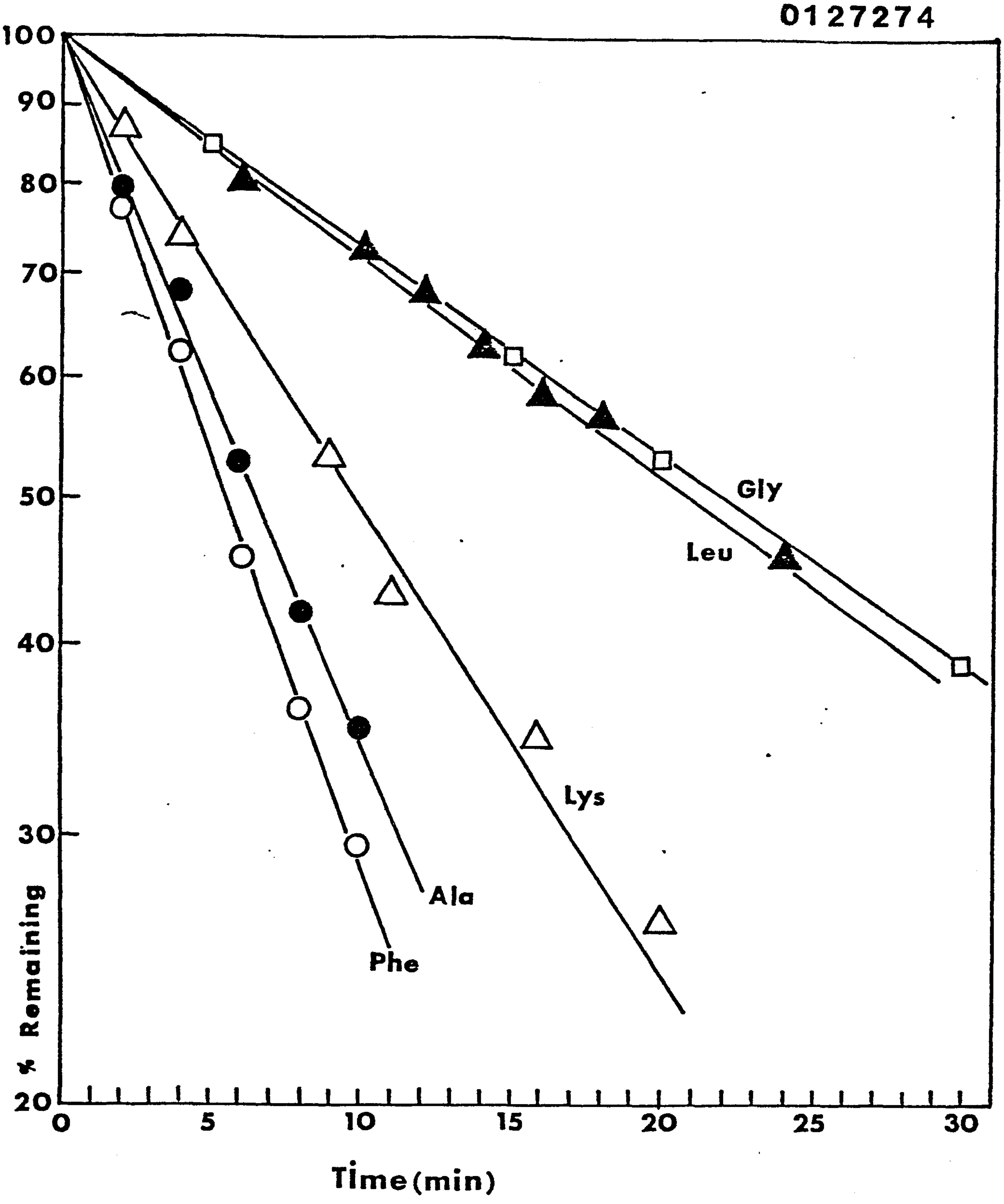


Fig. 1 - Human serum - catalyzed hydrolysis of amino-acid esters of metronidazole at 37 C. Actual data were normalized for easier comparison.

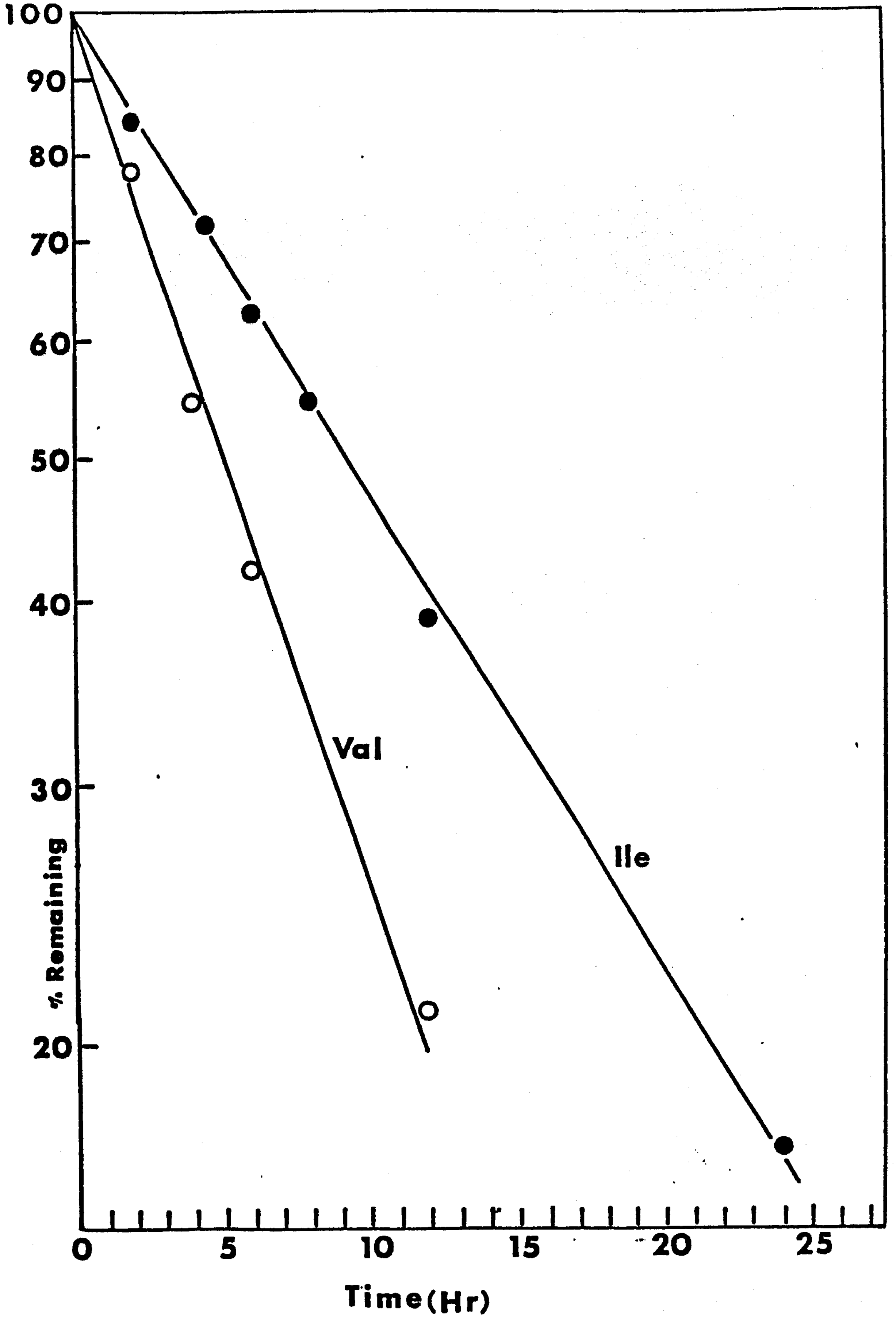


Fig. 2 - Human serum - catalyzed hydrolysis of Ile and Val esters of metronidazole at 37 C.